(19) Europäisches Patentamt
European Patent Office
Office européen des brevets

(11) **EP 2 092 929 B1**

(12) **EUROPÄISCHE PATENTSCHRIFT**

(45) Veröffentlichungstag und Bekanntmachung des Hinweises auf die Patenterteilung:
**17.02.2016 Patentblatt 2016/07**

(51) Int Cl.:
*A61K 8/26* (2006.01)     *A61K 8/35* (2006.01)
*A61K 8/41* (2006.01)     *A61K 8/49* (2006.01)
*A61Q 17/04* (2006.01)     *A61K 8/06* (2006.01)

(21) Anmeldenummer: **08003245.1**

(22) Anmeldetag: **22.02.2008**

(54) **Lichtschutzzubereitung in Form einer W/O-Emulsion mit einem Lichtschutzfaktor von _> 50**

Sun block preparation in the form of a w/o emulsion with a sun block factor of _> 50

Préparation de protection contre la lumière sous la forme d'émulsions eau/huile ayant un facteur de protection contre la lumière de _> 50

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**26.08.2009 Patentblatt 2009/35**

(60) Teilanmeldung:
**15202105.1**

(73) Patentinhaber: **STADA Arzneimittel AG**
**61118 Bad Vilbel (DE)**

(72) Erfinder:
• **Hansen, Dr. Peter**
**63303 Dreieich (DE)**
• **Heppner, Andrea**
**61197 Florstadt (DE)**
• **Rillmann, Dr. Thomas**
**76756 Bellheim (DE)**

• **Schumann, Christof**
**35767 Breitscheid-Erdbach (DE)**

(74) Vertreter: **Wittkopp, Alexander**
**Maiwald Patentanwalts GmbH**
**Jungfernstieg 38**
**20354 Hamburg (DE)**

(56) Entgegenhaltungen:
**EP-A- 1 074 241     EP-A- 1 281 390**
**EP-A- 1 310 235     WO-A-2005/053633**
**US-A1- 2005 118 211     US-A1- 2007 264 292**
**US-B1- 6 881 415**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft Lichtschutzzubereitungen in Form einer W/O-Emulsion, die einen Lichtschutzfaktor von ≥ 50 aufweisen und den anorganischen Konsistenzgeber Stearalkonium Hectorit enthalten.

Die schädigende Wirkung des ultravioletten Teils der Sonnenstrahlung auf die Haut ist seit langem bekannt. Derartige Schädigungen können eine einfache Hautreizung, z.B. ein leichter Sonnenbrand sein, können sich aber auch in Zellschädigungen manifestieren. Es ist bekannt, dass vor allem durch UV-B-Strahlung (das ist Strahlung mit einer Wellenlänge zwischen 290 nm und 320 nm) in der Haut DNS-Schäden hervorgerufen werden können, die zu Zellmutationen und somit zu Hautkrebs führen.

Auch werden die vorzeitige Hautalterung, die Bildung von Falten und die Erschlaffung des Hautbindegewebes durch UV-Strahlung beschleunigt; verantwortlich hierfür ist vor allem die langwellige UV-A-Strahlung (das ist Strahlung mit einer Wellenlänge zwischen 320 nm und 400 nm). Auch sie begünstigt die Auslösung einer Erythembildung oder verstärkt diese Reaktion bei manchen Personen, und sie kann sogar die Ursache für durch Licht ausgelöste toxische oder allergische Reaktionen sein.

Zur Vorbeugung gegen derartige Schädigungen sind in den letzten Jahren immer effektivere Sonnenschutzmittel entwickelt worden, in denen zahlreiche physikalische und chemische Filtersubstanzen die Haut vor UV-A-Strahlung und vor UV-B-Strahlung schützen sollen.

Physikalische Lichtschutzfilter, wie z.B. Oxide von Metallen, wirken auf der Haut verteilt wie kleine Spiegel, die die UV-Strahlung reflektieren und streuen.

Durch chemische Lichtschutzfilter wird die auf die Haut auftreffende UV-Strahlung in Wärmeenergie umgewandelt. Chemische Lichtschutzfilter können sowohl lipophile als auch hydrophile Eigenschaften aufweisen und entsprechend dieser Eigenschaften unterscheidet man zwischen öllöslichen und wasserlöslichen Filtern.

Lichtschutzzubereitungen werden oftmals als Emulsionen, Lotionen oder Gele formuliert.

Aufgabe der vorliegenden Erfindung war es, gegenüber dem Stand der Technik verbesserte Lichtzusammensetzungen anzugeben.

Insbesondere war es Aufgabe der vorliegenden Erfindung, Zusammensetzungen anzugeben, die als W/O-Emulsion formuliert, einen Lichtschutzfaktor von ≥ 50 aufweisen.

Durch umfangreiche Untersuchungen hat die Anmelderin in unerwarteter und überraschender Weise festgestellt, dass durch Zusatz des anorganischen Konsistenzgebers Stearalkonium Hectorit der Lichtschutzfaktor von W/O-Emulsionen mit einem geringen Anteil an UV-B-Filtern gesteigert werden kann und Lichtschutzfaktoren von ≥ 50 erzielt werden können.

Sie hat des Weiteren überraschend festgestellt, dass durch Kombination des anorganischen Konsistenzgebers Stearalkonium Hectorit mit PEG-freien W/O-Emulgatoren, insbesondere dem W/O-Emulgator Polyglyceryl-4, Diisostearate/Polyhydroxystearate/Sebacate ausgesprochen stabile, wasser- und seewasserfeste W/O-Lichtschutzformulierungen hergestellt werden können, die vorzugsweise einen Lichtschutzfaktor ≥ 50 aufweisen. Sie hat des Weiteren überraschend gefunden, dass stabile W/O-Lichtschutzformulierungen mit derartig hohen Lichtschutzfaktorwerten durch Kombination des anorganischen Konsistenzgebers Stearalkonium Hectorit mit PEG-freien W/O-Emulgatoren, insbesondere dem Polyglycery-4, Diisostearate/Polyhydroxystearate/Sebacate auch erzielt werden können, wenn keine PEG-Emulgatoren in diese Zusammensetzungen eingearbeitet werden.

Gegenstand der vorliegenden Erfindung ist zum einen eine W/O-Emulsion mit einem Lichtschutzfaktor ≥ 50, enthaltend den anorganischen Konsistenzgeber Stearalkonium Hectorit.

In einer bevorzugten Ausführungsform enthält die erfindungsgemäße Lichtschutzzubereitung lediglich geringe Gehalte an bei Raumtemperatur festen reinen UV-B-Filtern von insgesamt ≤ 10 Gew.-%, vorzugsweise ≤ 5 Gew.-%, besonders bevorzugt ≤ 2 Gew.-%.

In einer weiteren bevorzugten Ausführungsform enthält die erfindungsgemäße Lichtschutzzubereitung mindestens einen PEG-freien W/O-Emulgator, insbesondere den W/O-Emulgator Polyglyceryl-4, Diisostearate/Polyhydroxystearate/Sebacate.

Weiterhin bevorzugt enthält die erfindungsgemäße Lichtschutzzubereitung zusätzlich mindestens einen UV-A-Filter, bevorzugt Diethylamino Hydroxybenzoyl Hexyl Benzoate (Uvinul A Plus der Firma BASF) und/oder Butylmethoxydibenzoylmethane (Parsol 1789 von DSM bzw. Neo Heliopan 357 von Symrise) und/oder Bis-Ethylhexyloxyphenol Methoxyphenyl Triazine (Tinosorb S von der Firma Ciba) und/oder anorganische Pigmente, insbesondere Zinkoxid.

Es wurde überraschend gefunden, dass die Verwendung der UV-A-Filter Butylmethoxydibenzoylmethane und/oder Diethylamino Hydroxybenzoyl Hexyl Benzoate und/oder Bis-Ethylhexyloxyphenol Methoxyphenyl Triazine zu einer Steigerung der UV-B-Schutzwirkung der erfindungsgemäßen Lichtschutzzubereitungen führt.

Es wurde des Weiteren überraschend gefunden, dass erfindungsgemäße W/O-Emulsionen mit einem Lichtschutzfaktor von ≥.50, die den anorganischen Konsistenzgeber Stearalkonium Hectorit enthalten, einen UV-A-Schutzfaktor ≥ 20 und eine UV-A/UV-B-Ratio ≥ 1/3 aufweisen, wenn sie des Weiteren wenigstens einen UV-A-Filter, vorzugsweise den/die UV-A-Filter Butylmethoxydibenzoylmethane und/oder Diethylamino Hydroxybenzoyl Hexyl Benzoate und/oder Bis-Ethylhexyloxyphenol Methoxyphenyl Triazine enthalten.

Der UV-A-Schutzfaktor wird entweder nach der in-vivo-PPD-Methode bestimmt ("Persistent pigment darkening method in der von der französischen Gesundheitsagentur Agence francaise de securité sanitaire des produits de santé - Afssaps geänderten Form"), oder nach der in-vitro-COLIPA-Methode bestimmt ("In vitro UVA test method gemäß COLIPA Recommendation No. 20").

Die UV-A7UV-B-Ratio ist das Verhältnis des UV-A-Schutzfaktors zum UV-B-Schutzfaktor.

W/O-Emulsionen stellen übliche Formulierungsgrundlagen für Sonnenschutzzusammensetzungen dar, insbesondere wenn die Wasserfestigkeit der Zusammensetzungen im Vordergrund steht. Bisher war es aber nicht möglich, stabile W/O-Emulsionen mit einem Lichtschutzfaktor $\geq$ 50 herzustellen.

Der Lichtschutzfaktor ist die Maßzahl, mit der die Wirksamkeit einer Lichtschutzformulierung gemessen wird und gibt an, wie viel länger man sich mit der auf die Haut aufgetragenen Lichtschutzformulierung der Sonne aussetzen kann, als dies mit der jeweils individuellen Eigenschutzzeit möglich wäre, bis ein Sonnenbrand entsteht. Wird vor und nach Auftragen eines Lichtschutzpräparates die minimale Erythemdosis (MED), das heißt die Menge an UV-B-Bestrahlung, die ein gerade erkennbares Erythem induziert, bestimmt, ergibt sich der Lichtschutzfaktor (LSF) nach folgender Formel:

$$\text{LSF} = (\text{MED mit Sonnenschutzmittel}) / (\text{MED ohne Sonneschutzmittel})$$

Der Lichtschutzfaktor wird nach der "International Sun Protection Factor Method (2006)" bestimmt.

Als erfindungsgemäße Konsistenzgeber wird oder anorganische Konsistenzgeber Stearalkonium Hectorit eingesetzt.

Das Stearalkonium Hectorit kann erfindungsgemäß zusammen mit dem C12-15 Alkyl Benzoate oder Propylene Carbonate eingesetzt werden. Diese Kombination ist kommerziell beispielsweise unter der Bezeichnung Bentone Gel TN-V von Nordmann und Rassmann erhältlich.

Der anorganische Konsistenzgeber Stearalkonium Hectorit liegt in den erfindungsgemäßen W/O-Emulsionen bevorzugt in Mengen von 0,01 bis 40 Gew.-%, insbesondere von 0,1 bis 20 Gew.-%, besonders bevorzugt von 0,1 bis 2 Gew.-% vor.

Der zur Erzielung besonders stabiler und wasserfester Zusammensetzungen in Kombination mit dem anorganischen Konsistenzgeber eingesetzte W/O-Emulgator Polyglyceryl-4, Diisostearate/Polyhydroxystearate/Sebacate liegt in den erfindungsgemäßen Zusammensetzungen bevorzugt in Mengen von 0,1 bis 10 Gew.-%, insbesondere von 1 bis 6 Gew.-% vor.

Vorzugsweise liegt das Verhältnis von W/O-Emulgator, insbesonderen dem Polyglyceryl-4, Diisostearate/Polyhydroxystearate/Sebacate, zum anorganischen Konsistenzgeber Stearalkonium Hectorit in den erfindungsgemäßen Zusammensetzungen im Bereich von 0,03 bis 100, bevorzugt von 0,1 bis 30, besonders bevorzugt von 1 bis 30.

Die erfindungsgemäßen W/O-Lichtschutzzubereitungen enthalten wenigstens einen UV-B-Filter. Als bevorzugte UV-B-Filter werden die folgenden UV-Filter eingesetzt:

p-Aminobenzoesäurederivate, z.B. 4-Aminobenzoesäure, 4-Dimethylaminobenzoesäure-2-ethylhexylester (Eusolex 6007) oder ethoxyliertes Ethyl-4-aminobenzoat (Uvinul P25),

Salicylsäurederivate, z.B. 3,3,5-Trimethyl-cyclohexyl-salicylat (Neo Heliopan HMS) oder Salicylsäure-2-ethylhexylester (Neo Heliopan OS),

Benzophenonderivate, z.B. 2-Hydroxy-4-methoxy-benzophenon (Neo Heliopan BB) oder 2-Hydroxy-4-methoxy-benzophenon-5-sulfonsäure und ihr Natriumsalz (Uvinul MS 40),

Diphenylacrylate, z.B. 2-Cyano-3,3-diphenylacrylsäure-2-ethylhexylester (Neo Heliopan 303),

Campherderivate, z.B. 3-(4'-Trimethylammonium)-benzyliden-bornan-2-onmethylsulfat (Mexoryl SO), 3-(4'-Sulfo)-benzyliden-bornan-2-on und seine Salze (Mexoryl SL) oder 3-(4'-Methylbenzyliden)-DL-campher (Eusolex 6300) oder 3-Benzylidencampher (Mexoryl SDS20),

Triazinderivate, z.B. 2,4-Bis[4-(2-ethylhexyloxy)-2-hydroxyphenyl]-6-(4-methoxyphenyl)-1,3,5-triazin (Tinosorb S),

Benzotriazolderivate, z.B. 2-(2H-Benzotriazol-2-yl)-4-methyl-6-(2-methyl-3-(1,3,3,3-tetramethyl-1-(trimethylsilyloxy)disiloxanyl)propyl)phenol (Mexoryl XL),

Benzoxazol-Derivate, z.B. 2,4-Bis-[5-1(dimethylpropyl)benzoxazol-2-yl-(4-phenyl)-imino]-6-(2-ethylhexyl)-imino-1,3,5-triazin (Uvasorb K2A),

Polymer von N-[2(und 4)-(2-oxoborn-3-ylidenmethyl)benzyl]acrylamid (Mexoryl SW),

3-(4-(2,2-bis Ethoxycarbonylvinyl)-phenoxy)propenyl)-methoxysiloxan/Dimethylsiloxan-copolymer (Parsol SLX).

Erfindungsgemäß sind die organischen UV-Filter jeweils bevorzugt in Mengen von 0,1 bis 10 Gew.-%, vorzugsweise 0,5 bis 8 Gew.-%, insbesondere 1 bis 7 Gew.-% in den erfindungsgemäßen Zusammensetzungen enthalten.

Vorzugsweise sind die erfindungsgemäßen Lichtschutzzubereitungen, insbesondere wenn sie mindestens einen UV-A-Filter enthalten, frei von UV-Filtern des CinnamatTyps, insbesondere frei von 4-Methoxyzimtsäure-2-ethylhexylester (Neo Heliopan AV) und/oder 4-Methoxyzimtsäureisoamylester (Neo Heliopan E1000). Ganz besonders bevorzugt sind die erfindungsgemäßen W/O-Zubereitungen, enthaltend mindestens einen UV-A-Filter, frei von UV-Filtern des Cinnamattyps.

Die Anmelderin hat des Weiteren überraschend festgestellt, dass sich W/O-Emulsionen mit einem Lichtschutzfaktor ≥ 50 auch ohne Zusatz wasserlöslicher UV-Filter herstellen lassen. Gemäß einer weiteren bevorzugten Ausführungsform der Erfindung sind die erfindungsgemäßen W/O-Emulsionen mit einem Lichtschutzfaktor ≥ 50 frei von wasserlöslichen UV-Filtern.

Ebenso überraschend hat die Anmelderin festgestellt, dass sich W/O-Emulsionen mit einem Lichtschutzfaktor ≥ 50 auch ohne Zusatz von UV-B-Filtern des Triazintyps, das heißt von Triazinderivaten herstellen lassen. Gemäß einer ebenso bevorzugten Ausführungsform sind die erfindungsgemäßen Lichtschutzzubereitungen frei von UV-B-Filtern vom Triazintyp, das heißt von Triazinderivaten, wie z.B. 2,4,6-Tris[p-(2-ethylhexyl-oxycarbonyl)anilino]1,3,5-triazin (Uvinul T150) oder 4,4'-[(6-[4-((1,1-Dimethylethyl)aminocarbonyl)phenyl-amino]-1,3,5-triazin-2,4-diyl)diimino]bis(benzoesäure-2-ethylhexylester) (Uvasorb Heb).

Besonders überraschend hat die Anmelderin festgestellt, dass stabile W/O-Formulierungen mit derart hohen Lichtschutzwerten von ≥ 50 auch ohne Einarbeitung organischer Gelbildner, wie z.B. Acrylate oder Xanthan Gum erzielt werden können. Gemäß einer weiteren bevorzugten Ausführungsform sind die erfindungsgemäßen Lichtschutzzubereitungen frei von organischen Gelbildnern, insbesondere Acrylaten und/oder Xanthan Gum.

Die in den erfindungsgemäßen W/O-Lichtschutzzubereitungen enthaltenen UV-A-Filter sind vorzugsweise:

2-[4-(Diethylamino)-2-hydroxybenzoyl]-benzoesäurehexylester (Uvinul A plus),

Benzol-1,4-di(3-methyliden-10-camphersulfonsäure) (Mexoryl SX),

Dibenzoylmethanderivate, z. B. 1-(4-tert.-Butylphenyl)-3-(4-methoxy-phenyl)-propan-1,3-dion (Parsol 1789),

Benzimidazolderivate, z. B. 2,2'-(1,4-Phenylen)bis(1H-benzimidazol-4,6-disulfonsäure, Mononatriumsalz) (Neo Heliopan AP).

Die UV-A-Filter sind in den erfindungsgemäßen Zusammensetzungen jeweils bevorzugt in Mengen von 0,2 bis 10 Gew.-%, insbesondere von 0,5 bis 8 Gew.-% enthalten.

Des Weiteren können die erfindungsgemäßen Zubereitungen mindestens einen pigmentären UV-Filter enthalten, wobei es sich um anorganisch und/oder organische pigmentäre UV-Filter handeln kann.

Vorteilhafte anorganische pigmentäre UV-Filter sind Metalloxide, insbesondere Oxide des Titans oder Zinks. Wenn anorganische Pigmente enthalten sind, werden diese vorzugsweise in mikronisierter Form eingesetzt, vorzugsweise in mittleren Teilchengrößen < 100 μm, besonders bevorzugt in mittleren Teilchengrößen zwischen 10 nm und 200 nm. Sie sind dann bevorzugt in Mengen von 1 bis 25 Gew.-%, insbesondere 1 bis 10 Gew.-% enthalten. Dabei ist es besonders vorteilhaft, wenn die anorganischen Pigmente in hydrophober Form vorliegen, d.h., dass sie oberflächig wasserabweisend ausgestaltet sind. Dies kann z.B. dadurch erfolgen, dass die Pigmentteilchen mit einer hydrophoben Oberflächenschicht aus Silikon überzogen werden. Derartige Pigmente sind kommerziell erhältlich beispielsweise von der Firma Tayca unter der Handelsbezeichnung MT 100 T, MT 100 Z, von der Degussa unter der Bezeichnung T 805 oder von DSM unter der Bezeichnung Parsol TX.

Als vorteilhafter organischer pigmentärer UV-Filter wird erfindungsgemäß das Methylen Bis-Benzotriazolyl Tetramethylbutylphenol (2,2'-Methylen-bis(6-(2H-benzotriazol-2-yl)-4-(1,1,3,3-tetramethylbutyl)phenol), vorzugsweise in einer Teilchengröße von < 100 μm eingesetzt. Insbesondere wird pigmentäres Methylene Bis-Benzotriazolyl Tetramethylbutylphenol mit einer mittleren Teilchengröße von 0,2 μm eingesetzt, welches als 50 %-ige Dispersion von der Firma Ciba Spezialitätenchemie unter der Bezeichnung Tinosorb M erhältlich ist.

Die erfindungsgemäßen W/O-Emulsionen umfassen grundsätzlich eine Ölphase, Wasser, ggf. Alkohol sowie die erfindungsgemäße Wirkstoffkombination. Die Ölphase kann dabei aus Fetten, Ölen, gelösten Wachsen oder sonstigen lipophilen Bestandteilen gebildet werden, insbesondere auch öllöslichen UV-Filtern. Als Öle können vorteilhaft Paraffinöl, mittelkettige Triglyceride, wie beispielsweise Myritol 318, Octyldodecanol, Isopropylmyristat, Jojobaöl oder Kokosnussöl enthalten sein. Die wässrige Phase der erfindungsgemäßen Emulsionen wird üblicherweise aus Wasser, ggf. im Gemisch mit Alkohol gebildet.

Besonders bevorzugt sind die erfindungsgemäßen Zusammensetzungen frei von die Hautverträglichkeit potentiell negativ beeinflussenden Zusatzstoffen, insbesondere enthalten sie keine PEG-Emulgatoren, Konservierungsstoffe oder Duftstoffe. Vorzugsweise sind die Zubereitungen wasserfest bzw. seewasserfest.

Das folgende Ausführungsbeispiel soll die vorliegende Erfindung veranschaulichen, ohne sie zu beschränken. Die Inhaltsstoffe sind gemäß INCI-Nomenklatur bezeichnet worden.

Beispiel 1: W/O-Emulsion mit einem Lichtschutzfaktor $\geq$ 50

| INCI | g/100 g |
|---|---|
| Polyglyceryl-4, Diisostearate/Polyhydroxystearate/Sebacate | 4,00 |
| Cera Alba | 2,00 |
| Caprylic/Capric Triglyceride | 10,00 |
| C12-15 Alkyl Benzoate | 6,00 |
| Bis-Ethylenhexyloxyphenol Methoxyphenyl Triazine | 2,00 |
| Octocrylene | 5,00 |
| Ethylhexyl Salicylate | 5,00 |
| Butyl Methoxydibenzoylmethane | 5,00 |
| Diethylamino Hydroxybenzoyl Hexyl Benzoate | 5,00 |
| C12-15 Alkyl Benzoate, Stearalkonium Hectorite (10 %-ig), Propylene Carbonate | 12,00 |
| Tocopheryl Acetate | 0,50 |
| Titanium Dioxide, Silica, Dimethicone | 4,00 |
| Buylene Glycol | 7,20 |
| Magnesium Sulfate | 0,50 |
| Trisodium NTA, Aqua | 0,26 |
| Citric Acid | 0,06 |
| Aqua | 31,48 |

Die W/O-Emulsion gemäß des Ausführungsbeispiels wird wie folgt hergestellt:

In einem Ansatzkessel wird die Ölphase, bestehend aus den öllöslichen UV-Filtern, dem W/O-Emulgator, dem Bienenwachs, dem Triglycerid und dem Alkyl Benzoate sowie dem anorganischen Konsistenzgeber vorgelegt und darin das Titandioxid, das Silica und das Dimethicone dispergiert. Die Mischung wird erwärmt, homogenisiert und anschließend das Tocopheryl Acetate zugegeben.

In einem anderen Ansatzkessel wird die Wasserphase aus Wasser, EDTA, Butylene Glycol, Magnesium Sulfate, Citronensäure vorgelegt und erwärmt.

Die beiden Phasen werden in der Wärme unter Homogenisieren vereinigt und anschließend auf Raumtemperatur abgekühlt.

**Patentansprüche**

1. Lichtschutzzubereitung in Form einer W/O-Emulsion, **dadurch gekennzeichnet, dass** sie den anorganischen Konsistenzgeber Stearalkonium Hectorit, Diethylamino Hydroxybenzoyl Hexyl Benzoate sowie wenigstens einen UV-B-Filter enthält und einen Lichtschutzfaktor $\geq$ 50 aufweist, wobei das Diethylamino Hydroxybenzoyl Hexyl Benzoate in Mengen von 0,2-10 Gew.-% enthalten ist.

2. Lichtschutzzubereitung gemäß Anspruch 1, **dadurch gekennzeichnet, dass** das Stearalkonium Hectorit in Mengen von 0,01 bis 40 Gew.-%, insbesondere von 0,1 bis 20 Gew.-% vorliegt.

3. Lichtschutzzubereitung gemäß Anspruch 1 oder 2, **dadurch gekennzeichnet, dass** sie zusätzlich einen PEG-freien W/O-Emulgator, insbesondere den W/O-Emulgator Polyglyceryl-4, Diisostearate/Polyhydroxystearate/Sebacate enthält.

4. Lichtschutzzubereitung nach Anspruch 3, **dadurch gekennzeichnet, dass** der PEG-freie W/O-Emulgator in Mengen von 0,1 bis 10 Gew.-%, insbesondere von 1 bis 6 Gew.-% vorliegt.

5. Lichtschutzzubereitung nach einem der Ansprüche 3 oder 4, **dadurch gekennzeichnet, dass** das Verhältnis von W/O-Emulgator zum anorganischen Konsistenzgeber im Bereich von 0,03 bis 100, insbesondere von 0,1 bis 30, besonders bevorzugt von 1 bis 30 liegt.

6. Lichtschutzzubereitung gemäß einem der Ansprüche 1 bis 5, **dadurch gekennzeichnet, dass** sie einen UV-A-Schutzfaktor von $\geq$ 20 aufweist.

**Claims**

1. Light protection preparation in the form of a W/O-emulsion, **characterized in that** it contains the inorganic consistency enhancer stearalkonium hectorite, diethylamino hydroxybenzoyl hexyl benzoate and at least one UV-B-filter, and having a light protection factor $\geq$ 50, whereby the diethylamino hydroxybenzoyl hexyl benzoate is contained in an amount of 0.2-10% by weight.

2. Light protection preparation according to claim 1, **characterized in that** the stearalkonium hectorite is contained in an amount of 0.01 to 40% by weight, preferably 0.1 to 20% by weight.

3. Light protection preparation according to claim 1 or 2, **characterized in that** it additionally contains a PEG-free W/O-emulsifier, preferably the W/O-emulsifier polyglyceryl-4, diisostearatelpolyhydroxystearatelsebacate.

4. Light protection preparation according to claim 3, **characterized in that** the PEG-free W/O-emulsifier is contained in an amount of 0.1 to 10% by weight, preferably 1 to 6% by weight.

5. Light protection preparation according to one of claims 3 or 4, **characterized in that** the ratio of W/O-emulsifier to the inorganic consistency enhancer is in the range from 0.03 to 100, preferably 0.1 to 30, more preferably 1 to 30.

6. Light protection preparation according to one of claims 1 to 5, characterrized in that it has a UV-A-protection factor of $\geq$ 20,

**Revendications**

1. Préparation photoprotectrice sous la forme d'une émulsion W/O, **caractérisée en ce qu'**elle contient un épaississeur inorganique de stearalkonium hectorite, diethylamino hydroxybenzoyl hexyl benzoate et au moins un filtre UV-B, et ayant un facteur de protection solaire $\geq$ 50, le Diethylamino hydroxybenzoyl hexyl benzoate étant contenu en une quantité de 0.2-10% en masse.

2. Préparation photoprotectrice selon la revendication 1, **caractérisée en ce que** le stearalkonium hectorite est contenu en une quantité de 0,01 à 40% en masse, de préférence 0,1 à 20% en masse.

3. Préparation photoprotectrice selon la revendication 1 ou 2, **caractérisée en ce qu'**elle contient en plus un émulsifiant W/O libre de PEG, de préférence l'émulsifiant W/O de polyglyceryl-4, diisostearate/polyhydroxystearate/sebacate.

4. Préparation photoprotectrice selon la revendication 3, **caractérisée en ce que** l'émulsifiant W/O libre de PEG est contenu en une quantité de 0,1 à 10 % en masse, de préférence 1 à 6% en masse.

5. Préparation photoprotectrice selon l'une des revendications 3 ou 4, **caractérisée en ce que** le rapport de l'émulsifiant W/O à l'épaississeur inorganique est dans la gamme de 0,03 à 100, de préférence 0,1 à 30, de manière plus préférée 1 à 30.

6. Préparation photoprotectrice selon l'une des revendications 1 à 5, **caractérisée en ce qu'**elle a un facteur de protection UV-A $\geq$ 20.